# EUROPEAN PATENT APPLICATION

(11) **EP 4 530 706 A1**
(43) Date of publication of application: **02.04.2025**
(21) Application number: 23306603.4
(22) Date of filing: 26.09.2023
(51) Int. Cl.: G02C 11/00, G06N 20/10, A61B 5/00, G06N 3/02

(54) **WEARING DETECTION MODULE TO BE FIXED ON A SPECTACLE FRAME AND PROCESS FOR DETERMINING WHETHER A SPECTACLE FRAME IS WORN**

(71) Applicant: Essilor International, 94220 Charenton-Le-Pont (FR)
(72) Inventor: LE CAIN, Aurélie, 94000 Créteil (FR); PIC, Eléonore, 94300 Vincennes (FR); FOLIGNONI, Cédric, 94220 Charenton le Pont (FR); RATNARAJAH, Ethan, 95170 Deuil-la-Barre (FR)
(74) Representative: Jacobacci Coralis Harle

(57) **Abstract**

The invention relates to a process for determining the wearing status of a wearable device (120) performed by a processing unit (141), said process comprising steps of:
- measuring a n-tuple of values of several physical parameters each suitable for estimating whether the wearable device (120) is worn by a wearer or not, and
- determining if the wearable device (120) is worn as a function of said n-tuple of values,
characterized in that, several n-tuples of values of said physical parameters being stored in a database and being classified in at least two clusters, during the step of determining, it is determined if the measured n-tuple of values belongs to one or another of said clusters and it is deduced therefrom whether the wearable device (120) is worn or not.

## Description

### TECHNICAL FIELD OF THE INVENTION

The invention relates to a device and a process for determining whether a wearable device (for instance a spectacle frame) is worn or not.

It more particularly relates to the optical domain, but not only.

The invention can have various applications, for instance (but not imitatively):
- the monitoring of a wearing time of an eyewear having lenses suitable to slow down the evolution of myopia,
- the battery optimization (for instance by automatically turn on or off some sensors embedded in an eyewear depending on their usefulness),
- the pleasantness of a listening device paired to an eyewear (for instance by automatically stopping a podcast or a track when an eyewear is removed and restarting it at the precise moment when the eyewear is put on),
- the data storage optimization (for instance by registering data only when the eyewear is worn),
- the monitoring of whether an eyewear is correctly worn (more particularly if this eyewear comprise progressive addition lenses).

### BACKGROUND INFORMATION AND PRIOR ART

More and more electronic spectacle frames are being develop with a great variety of different electronic elements. This is for instance the case for the spectacle frames of myopic wearers.

Myopia has increased worldwide during recent years and is becoming a serious public problem. For instance, in East Asia, the prevalence can reach 80% of the population.

In this context, many techniques have been developed to stop or slow down the evolution of myopia. Among these techniques, several strategies based on special corrective eyeglasses have been reported as effective.

One of the important parameters to reduce this evolution is to wear the special corrective eyeglasses very regularly. But this regularity is difficult to control in children, especially when they can still see well enough to play without their glasses. Assessing this parameter of wearing time is crucial to predict the outcome of the treatment.

In this context, document US2018081201 discloses a pair of sensors able to determine when the spectacles are being worn by a child. These sensors are for instance arranged, in use, to measure the temperature in the inside face of the spectacle frame relative to the ambient temperature. When both sensors detect that the temperature in the inside face is greater than the sum of the ambient temperature and a predetermined threshold, it is considered that the frame is worn by the patient.

Nevertheless, the provided information is not reliable enough. Indeed, many false positives can be generated, such as when the user holds the spectacle frame in his hand.

Therefore, there is a need for a solution allowing to detect in a more robust manner if the spectacle frame is really worn on the head of the wearer.

### SUMMARY OF THE INVENTION

In this context, the present invention provides a process for determining the wearing status of a wearable device performed by a processing unit, said process comprising steps of:
- measuring the value of at least one physical parameter suitable for estimating whether the wearable device is worn by a wearer or not, each measured value forming a n-tuple with n greater than or equal to 1, and
- determining if the wearable device is worn as a function of said n-tuple of values,
wherein said step of determining is carried out thanks to a machine learning model.

In a preferred embodiment, several n-tuples of values of said physical parameter(s) being stored in a database and being classified in at least two clusters, during the step of determining, it is determined if the measured n-tuple of value(s) belongs to one or another of said clusters and it is deduced therefrom whether the wearable device is worn or not. Here, we note that the model used to generate these clusters is not necessarily a clustering model, but it can be any other kind of machine learning model able to differentiate at least two kinds of wearing states.

The use of such an algorithm to determine the worn state has many advantages.

The first one is that the results do not depend on the initial calibration of the sensors, on possible mechanical problems that can occur (for example, addition of an offset due to the displacement of a sensor), and on possible variations (for instance, during winter, the temperature gap inside and outside the pair of eyeglasses 110 is generally greater than during summer). Consequently, this solution gives better results in the sense that the results can be corrected in real time by updating regularly and automatically the clusters.

Another advantage is that, if the quality of clustering has to be improved, it is possible to refresh the database by removing old data in order to run the algorithm on the basis only of the most recent data. This can be carried out without human intervention.

In a preferred embodiment, this process is performed so as to determine if the wearable device is worn, not worn or worn but not well-fitted. Consequently, it is possible to detect changes in the way of wearing the frame and the wearer can be warned if there is a difference in the resting worn position. This is especially the case when the wearer is wearing progressive addition lenses because the height of the pupil is an important parameter of comfort and good vision.

Other preferred features of the invention are the following ones:
- n is greater than or equal to 2.
- several n-tuples of value(s) of said physical parameter(s) are measured during time, and it is determined whether the wearable device is worn or not as a function of the variation of said values during time.
- a rolling mean or standard deviation of said measured n-tuples of value(s) is calculated and it is determined whether the wearable device is worn or not as a function of the variation of said rolling mean or standard deviation.
- the measured n-tuple of values is stored in said database and a clustering algorithm is then performed by said processing unit on all the n-tuples of values stored in said database to generate new clusters.
- said clustering algorithm is based on a K-means method or a gaussian mixture method.
- said n-tuples of values are classified in at least three clusters associated to a worn state, a non-worn state, and a worn but non-fitted state.
- at least two of said physical parameters are not of the same nature.
- in a variant, at least two of said physical parameters are of the same nature.
- at least one of said parameters relates to: a position of a temple of the frame relative to another part of the frame, or a contact area or a distance between the wearable device and the wearer, or a light emitted and reflected back by the wearer head, or a temperature on an internal face of the wearable device, or an inclination of the wearable device relative to the ground.
- in a first embodiment, the database is initially empty and is gradually filled with the measurements.
- in a second embodiment, the database is initially filled with values that are labeled by a worn state.
- in this second embodiment, the database is not filled with the measurements (n-tuples of values).
- in a variant, in this second embodiment, the database is filled with at least some of the measurements.
- in a variant, in this second embodiment, a learning operation is carried out to fill the database with measurements labelled by a worn state,
- in a variant, said machine learning model is trained on the basis of a database that stores, in registers, n-tuples of values and, associated to each n-tuple, a wearing state, said database being initially filled with first registers issued from values measured on individuals others than said wearer and being then completed with new registers issued from values measured on said wearer,
- preferably, the wearing state in the new registers are determined as a function of the data stored in the first registers,
- in another variant or in addition, the wearing state in the new registers are determined by an individual during a training period, for instance by means of a button thanks to which the individual can indicate when the wearable device is worn or not (said individual being distinct from the wearer or not).

The invention also relates to a wearing detection module configured to be fixed on a wearable device, comprising:
- several wearing sensors configured to measure a n-tuple of value(s) of at least one physical parameter, each parameter being suitable for estimating whether the wearable device is worn by a wearer or not, and
- a processing unit programmed to receive said n-tuple of value(s) and to determine whether the wearable device is worn or not, as a function of said n-tuple of value(s),
wherein said processing unit is programmed to determine whether the wearable device is worn or not thanks to a machine learning model.

In a preferred embodiment, several n-tuples of values of said parameter(s) being stored in a database and being classified in at least two clusters, the processing unit is programmed to determine whether the wearable device is worn or not by determining if the measured n-tuple of values belong to one or another of said clusters.

In a preferred embodiment, distinct n-tuples of value(s) of said physical parameters being measured during time, the processing unit is programmed to determine whether the wearable device is worn or not as a function of the variation of said values during time.

In a preferred embodiment, the measured n-tuple of value(s) is stored in said database and a clustering algorithm is then performed by said processing unit on the n-tuples of value(s) stored in said database to generate new clusters.

In a preferred embodiment, when the wearing detection module is new and ready to be sold, said database already stores several n-tuples of value(s) of said parameters.

In a preferred embodiment, the module comprises a battery configured to power the processing unit and the wearing sensors.

The invention also relates to a monitoring equipment comprising:
- a wearable device, and
- a wearing detection module as defined above, fixed on said frame.

In a preferred embodiment, said wearable device is a frame of an eyewear that includes at least one lens that is fixed to the frame and that is able to change the natural evolution of an optical deficiency.

In a preferred embodiment, said wearable device is a frame of an eyewear and wherein said wearing sensors are embedded in a temple of said frame.

### DETAILED DESCRIPTION OF EXAMPLE(S)

The following description with reference to the accompanying drawings, given by way of non-limiting example makes it clear what the invention consists in and how it can be reduced to practice.

In the accompanying drawings:
- Figure 1 is a schematic view of a monitoring equipment according to the invention,
- Figure 2 is a graphic illustrating points the coordinates of which are determined by means of two sensors fitted in the monitoring equipment of Figure 1,
- Figure 3 is a graphic illustrating variations of values during time t, these values being determined by means of sensors fitted in the monitoring equipment of Figure 1,
- Figure 4 illustrates a process for determining the wearing status of a spectacle frame comprising the monitoring equipment of Figure 1.

The invention relates to a wearable device including means for determining how long this wearable device has been worn.

This wearable device is preferably a head-mounted device. It is more preferably a spectacle frame.

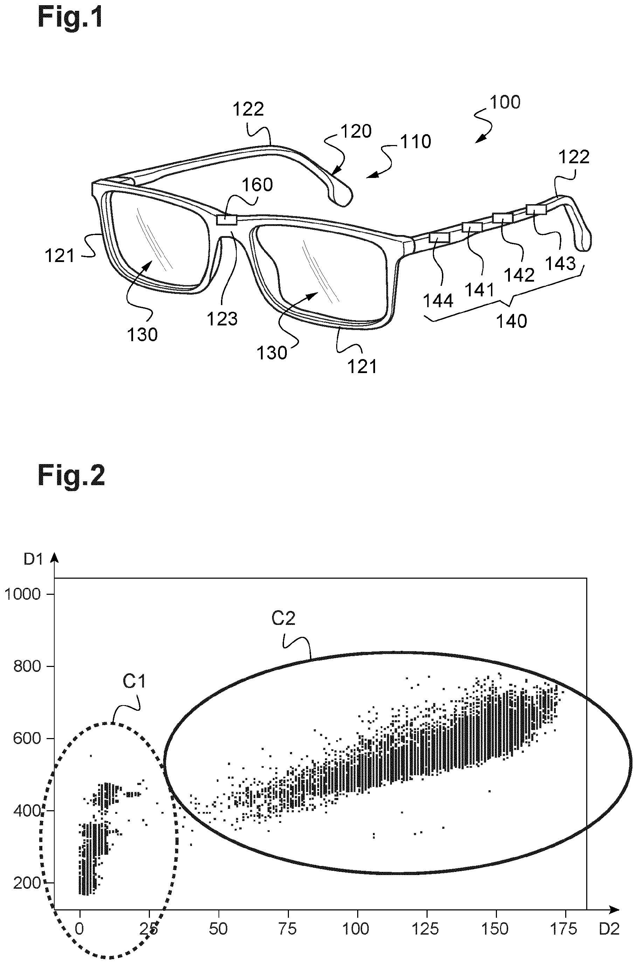

In the embodiment shown in Figure 1, the wearable device belongs to a monitoring equipment 100 that comprises an eyewear having at least one lens designed to change the natural evolution of an optical deficiency.

In the following description, the considered optical deficiency will be myopia but the invention can also apply to other types of visual defects requiring the wearing of an eyewear to prevent a deterioration of vision quality, such as visual fatigue, jet lag, phototoxicity, age related macular degeneration.

The invention also applies to other domains. For instance, it can be used to assess the impact of the progressive lenses wearing time on a new wearer or the impact of light dose arriving on the eyes of a wearer. It can also be used to reduce the battery consumption of an electronic component fitted in a frame (by switching it off when the frame is not worn).

The monitoring equipment 100 is a specific combination of the eyewear and a wearing detection module 140 fixed on the eyewear.

According to the invention, this wearing detection module 140 comprises at least two wearing sensors 142, 143 configured to measure the values of two physical parameters (each parameter being suitable for estimating whether the spectacle frame is worn by a wearer) and a processing unit 141 configured to receive said values and to determine whether the eyewear is worn as a function of said values.

As used herein, the term "eyewear" refers generally to items and accessories worn on or over the eyes, which may be for purpose of improving or enhancing visual acuity or preventing myopia.

As shown in Figure 1, the eyewear is a pair of eyeglasses 110 that includes a frame 120 (that forms to the "wearable device"), and two lenses 130 fixed to the frame. In a variant, this eyewear could have a different shape (goggles, etc).

The frame 120 is a structure receiving, retaining, holding, and/or supporting the lenses such that these lenses 130 can be placed in front of respective eyes of a wearer when the pair of eyeglasses 110 is being worn.

As shown in this figure, the frame 120 includes two rims 121 for receiving the lenses 130. Here, each of the two rims 121 is a full rim. According to various embodiments, the rims may also be half rims, or the frame 120 may be without rim (the lenses being screwed onto the frame). Accordingly, the frame 120 may be a full rimmed frame or a semi-rimless frame or a rimless frame.

The frame 120 includes a bridge 123, situated above the nose of the wearer when the pair of eyeglasses 110 is being worn.

The frame 120 also includes a pair of frame temples 122. The pair of frame temples 122 constitutes a pair of elongated side pieces of the frame 120, rested on the ears of the wearer when the pair of eyeglasses 110 is being worn. Each temple 122 is articulated to the corresponding rim by a hinge.

In this very particular embodiment, the lenses 130 are designed to modify the natural evolution of an optical deficiency, here myopia. But the invention also applies to corrective and non-corrective lenses, including single vision or multi-vision lenses, as well as other elements used to correct, protect, or enhance vision.

The wearing detection module 140 is designed to determine if the pair of eyeglasses 110 is being worn by the wearer or not.

This module is preferably embedded in the frame. It is for instance molded into a part of the frame 120 (for instance in one of its temples).

In a variant, this wearing detection module 140 may be attached to the frame. It could be permanently attached to the frame, for instance by means of glue. Or it could be removably attached to the frame, for example by means of snap-fastening means or by Velcro^{®}.

This module is designed to frequently detect a wearing state (a state "worn" or "not worn").

As explained above, it comprises preferably at least two wearing sensors 141, 142 each that could be able to determine a "wearing state approximation". As explained under, in a variant, it could comprise only one sensor.

Each wearing sensor can be of any kind. They can be designed to measure the same kind of parameter (values of temperature, values of distance...). But in a variant, the two sensors do not measure the same kind of parameter (they are not of the same nature). In any case, the sensors are designed to perform different measurements so as to be able to differently determine the wearing state approximations.

In a first example, one of the wearing sensors measures a parameter indicative of the distance between the spectacle frame (the temple) and the wearer.

This sensor is for instance a light sensor, for example an infrared proximity sensor (a photodiode) configured to emit light and to detect the reflected light. An infrared proximity sensor placed on the temple 122 or on the nose pad 132 or on a rim 121 of the spectacle frame can indicate if the pair of eyeglasses 110 is probably worn or not. If not, no light is reflected.

In a variant, the sensor is a capacitive sensor, configured to measure the contact area or the distance between the spectacle frame and the wearer.

In a second example, one of the wearing sensors measures a temperature or a temperature difference.

For example, the sensor can comprise two cells suitable for measuring the temperature in two opposite directions (inside the temple and outside the temple). The detection of a difference of temperature between the two cells is a signature of the fact that the pair of eyeglasses 110 is probably worn.

In a third example, one of the wearing sensors is a resistive sensor configured to measure biological and/or physiological parameters of the wearer. For example, the resistive sensor may be a heart rate sensor capable of detecting heart beats in a part of a spectacle frame element close to the skin of the wearer. Such a resistive sensor has the advantage to be specific by discriminating living things and objects. The heart rate sensor is preferably arranged on the temple, at the extremity of the tip of the temple facing a temporal bone of the wearer or on the bridge.

In a fourth example, one of the wearing sensors is a motion sensor, for example an accelerometer adapted to determine if the frame moves or not. The detection of a constant movement is a signature of the fact that the pair of eyeglasses 110 is probably worn.

In a variant, this is an IMU (for "Inertial Motion Unit) including an inclinometer (and/or a gyroscope) that is able to determine the tilt of the frame relative to the ground so as to determine if this tilt shows that the pair of eyeglasses 110 is probably placed on a furniture or if it is probably worn.

In a fifth example, one of the wearing sensors is a pressure sensor adapted to determine if the frame temples bear against the wearer's head or not.

In a sixth example, one of the wearing sensors is a pH sensor adapted to determine if the frame is in contact with the wearer's head or not.

In a seventh example, one of the wearing sensors is a capacitive sensor adapted to detect the wearer's head.

Such a sensor is mainly composed of 2 parts, the electronic components and a copper layout laying down onto a semi-rigid tab. The electronic components are mostly used to manage the analog signal through the copper area and the digital communication with the processing unit. The copper layout acts as an antenna: it sends and receives an electrical field that is modulated by the proximity of the target (here, the wearer's head). In situation, the tab is integrated into the temple and the rigid part of the sensor is glued to the inside of the temple.

In an eighth example, one of the wearing sensors is adapted to detect whether the temples are in a closed state (against the rims of the frame) or in an opened state. Such a sensor will be called hereinafter as an "opening/closing sensor". Two variants can be contemplated to design such a sensor.

In a first variant, the sensor is positioned on a face of an electronic board facing a magnet located into the hinge of the corresponding temple. Both parts are designed in such a way that an opening detection by the sensor occurs at temple opening mid race and the opposite way for closure detection.

In a second variant, the electronic board can be located near the center of the temple and the magnet can either be located onto the opposite temple or into a rim or into the nose bridge 123.

In the here considered embodiment, the pair of eyeglasses 110 comprises exactly two sensors.

A first of the sensors 142 is a photodiode located in a frame temple 122 and configured to measure a distance between the frame temple 122 and the corresponding wearer head temple (when the frame 120 is being worn).

The second sensor 143 is also a photodiode located in the nose pad 123 of the frame, configured to measure a distance between this nose pad and the wearer head face (when the frame 120 is being worn).

The frame 120 is preferentially fitted with means for accumulating or generating an electrical energy to supply the processing unit 141 with an electrical current.

These means comprise here a battery 144.

In a variant, these means can comprise an energy harvesting transducer that converts some form of ambient energy into electricity. This transducer can for instance generate an electrical current from movements of the frame or from sun light.

The processing unit 141 is programmed to:
- acquire a value determined by each wearing sensor 142, 143,
- deduce therefrom whether the spectacle frame is worn.

This enables to determine the length of time the wearer has worn the pair of eyeglasses 110 during a predetermined period, and, if any, to compare said length of time with at least a threshold to determine a level of efficiency of the optical deficiency treatment.

To this end, the processing unit 141 comprises a central processing unit (CPU), a memory and input/output components.

Thanks to its memory, the processing unit stores information used in the process described below. It particularly stores a computer application, consisting of computer programs comprising instructions, the execution of which allowing the implementation by the processing unit 141 of the method described below.

Thanks to its input/output components, the processing unit 141 can receive data measured by the wearing sensors 142, 143.

It can also receive other information from other sensors.

In the embodiment shown in Figure 1, this processing unit 141 is a microcontroller embedded in the pair of eyeglasses 110. In a variant, this processing unit could be remoted from the pair of eyeglasses 110 and formed by a controller of a computer or of a mobile device such as a smartphone.

Here, the frame 120 also comprises a communication unit 160 suitable to establish a radio contact with an informatics device (computer, smartphone, watch...). The processing unit 141 can therefore send information (for instance the length of time), so that this information can be processed by the informatics device.

This communication unit 160 is preferably a passive or active RFID chip. Such a chip does not require any battery to operate and can be scanned by a reader at a distance of 15 meters away from the scanner. In a variant, other technologies could be used (Bluetooth^{®}, WIFI...).

Here, the frame also comprises a clock (a Real Time Clock) embedded in the frame 120 and that is able to date the measure and a memory to memorize the last measures.

Consequently, the processing unit 141 can evaluate if the pair of eyeglasses 110 is worn or not and a simple log can register both the worn state and the corresponding time.

The process performed by this processing unit 141 to determine whether the pair of eyeglasses 110 is worn is executed in loops, at determined time intervals (also called "sampling period").

The time interval is preferably the same from a loop to another. It is advantageously greater than 5 seconds, to save electrical power. Here, a loop is done every 30 seconds.

To achieve this same aim, the execution of the process may be limited to a predetermined period (for instance from 6am to 11pm). A wearer is indeed considered as sleeping between 11pm and 6am. During the night, the processing unit 150 is consequently on standby.

In a variant or in addition, the execution of the process may be put on standby when the battery 144 is charging.

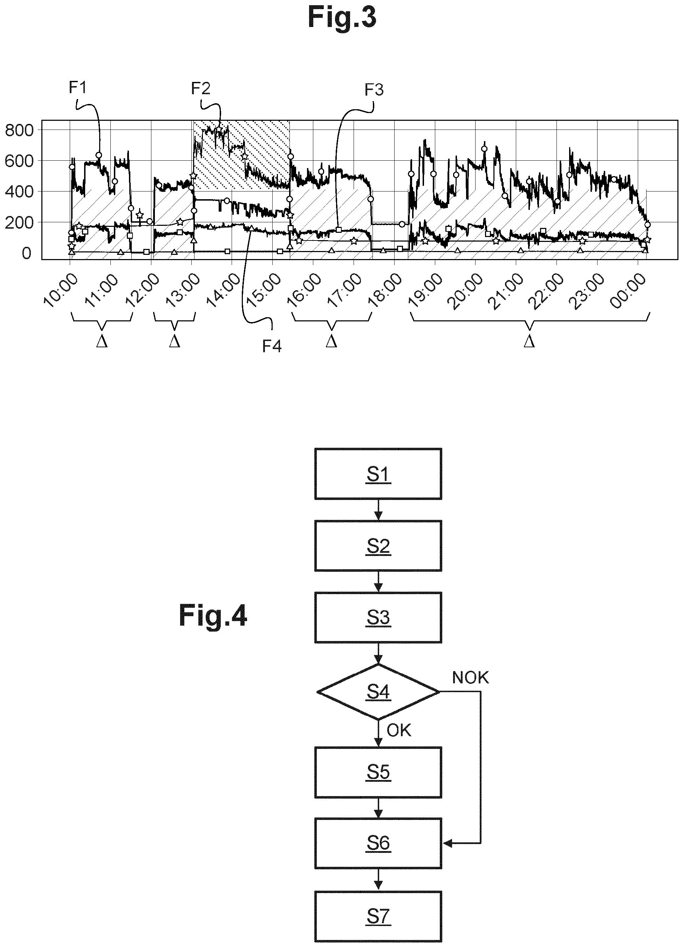

The process is carried out step by step, as shown in Figure 4.

The first step S1 consists in waking up the sensors.

The second step S2 consists in determining a measured first value D1 by means of the first sensor 142. This first measured value is such that the processing unit could calculate a first wearing state approximation ("a priori worn" or "a priori not worn"), but here, this calculation is not necessary.

In practice, in step S2, the distance between the frame temple and the possible wearer head temple (if any) is measured.

During the third step S3, the processing unit 141 determines a second value D2 by means of the second sensor 143, this second measured value being such that the processing unit could calculate a second wearing state approximation ("a priori worn" or "a priori not worn"), but here again, this calculation is not necessary.

In practice, in step S3, the distance between the frame nose pad and the possible wearer head face (if any) is measured.

In this embodiment, the first and second values are simultaneously measured.

In a variant, the second sensor may be waked up only if the first value has a degree of precision or of uncertainty less than a predetermined threshold, to prevent unnecessary energy loss.

At the end of this third step S3, because only two sensors are used, the processing unit 141 has determined a pair of instant values (named herein after the last pair of values).

More generally, if the eyeglass frame comprises a number n of sensors greater than 2, the aim at the end of step S3 for the processing unit 141 is to determine a n-tuple of values.

Then, during a fourth step S4, the process uses machine learning to determine the worn state.

To this end, various embodiments can be contemplated.

In every embodiment described hereafter, the processing unit 141 uses a database.

This database comprises at least two fields (or columns), a first one associated to the values measured by the first sensor 142 and the second one associated to the values measured by the second sensor 143. This database can be filled with registers, each one corresponding to a couple of values.

In a first embodiment, the process performed by the processing unit 141 is said non-supervised.

In this first embodiment, the database is initially empty (when the pair of eyeglasses is sold or lent to the future wearer).

Then, each time a pair of values is measured by the sensors, this pair is stored in a new register of the database.

Step S4 is implemented only when the database contains enough registers (for example more than 24 hours of recording).

During this step S4, the processing unit 141 is programmed to determine whether the pair of eyeglasses 110 is currently worn or not as a function of the last pair of measured values, and only on the basis of the old pairs of values stored in the database.

To this end, during this fourth step S4 or previously (for instance once a day), a first operation consisting in fitting the machine learning model is performed (here by cluster analysis). Such an analysis identifies commonalities in the stored pairs of values.

Then, during step S4 (when the last pair of values has been measured by the sensors), a second operation consisting in applying the model to this last pair of values is performed. This operation reacts based on the presence or absence of such commonalities in these new pair of values.

These operations can be detailed.

During the first clustering operation, the analysis of the registered data is made in two-dimensions because only two sensors are fitted in the pair of eyeglasses 110. It could be performed in a higher dimension when more than two sensors are used.

Several well-known algorithms can be used. One of them is known under the reference "k-means clustering".

This is a method of vector quantization that aims to partition n observations (that is to say n pairs of values measured by the two sensors) into k clusters. In this method, each observation belongs to the cluster with the nearest mean. This minimizes within-cluster variances and maximize without-cluster variances.

Here, the number k is preferably equal to two. Consequently, one cluster is associated to the worn state when the other is associated to the non-worn state. But in a variant, as it will be explained after, k may be greater than two.

In practice, the k-means clustering algorithm is performed on all the database registers. By considering that each pair of previously measured values form a point in a 2D representation (see Fig.2), this algorithm enables to distinguish two sets of points (the clusters C1 & C2), one set corresponding to data measured when the pair of eyeglasses 110 was worn and another set corresponding to data measured when the pair of eyeglasses 110 was not worn.

To associate one cluster with one of the worn states, distinct embodiments could be used. Here the selected solution consists in considering that the cluster that comprises the greatest number of points measured during night and/or during the battery charge corresponds to the non-worn state.

As explained above, a greater number of clusters can be used in order to distinguish other states than "worn" and "not worn". For instance, with k equal to three, a third cluster can be associated to a "sliding state", that is to say to a state where the pair of eyeglasses 110 is worn but has slipped along the nose of the wearer (in a bad position that has to be corrected).

At the end of this first clustering operation, the clusters are distinguished from each other by the processing unit 141.

During the second operation, the model is applied to the last pair of values.

To this end, in this embodiment, it is determined whether the point corresponding to this last pair of measured values belongs to one cluster or to the other. Specifically, it is determined if this point is nearer from one or the other of the centers of the clusters.

Then, if the nearest cluster corresponds to the worn state, the frame is considered worn, else, it is considered not worn.

In a variant of this first embodiment, the database may not be initially empty. For instance, when the pair of eyeglasses is sold or lent to the wearer, the database may already be filled with registers issued from databases of other eyewear or resulting from various tests performed during the design of the monitoring equipment 100. Then, the first and second operations can be carried out in the same way as mentioned above, by considering these registers.

According to a variation of this variant, the processing unit 141 can be programmed not to modify the database with new measured values, the list of recorded registers being unchanging (or being possibly editable only by an after-sales service). In this variation, the first clustering operation is performed once. This variation is not preferred because it needs to label a great number of pairs of values to obtain accurate results.

In a second embodiment, this step S4 is performed in a supervised manner.

In this embodiment, the processing unit 141 embedded in the eyewear does not store any database, but it is suitable to be wirelessly connected to a remote database.

This remote database comprises, as in the first embodiment, first and second fields to store a pair of values, and it further comprises a third field to store the worn state. Each registered observation (ie each pair of values stored in the database) is consequently labeled by an associated worn state (worn or not-worn).

Three examples for carrying out this step S4 will be described hereunder.

In all these examples, initially (when the pair of eyeglasses is sold or lent to the future wearer), the remote database is already filled with registers. These registers correspond to the results of various tests performed during the design of the monitoring equipment 100. These registers are for instance selected to comprise data as representative as possible of the values that could be measured by the two sensors in a great number of distinct environments, for various kinds of wearers behaviors.

Moreover, in these examples, a remote model is initially determined and fine-tuned according to the data comprised in this remote database. Every kind of model can be used (decision tree, neural network, etc.). Thanks to this model, weights can be calculated. These weights, once applied to a measured pair of values, indicate whether the eyewear is worn or not. They are stored in the processing unit 141 so that the latter can determine, at each sampling period, it the eyewear is worn.

In a first example, once the pair of eyeglasses sold or lent to the wearer, the processing unit 141 is programmed, when a pair of values is measured, to apply to these values the weights in order to deduce whether the eyewear is worn or not.

In a second example, once the pair of eyeglasses sold or lent to the wearer, the processing unit 141 is programmed to send new registers to the remote database, and the remote model is adjusted according to these new registers to take into account the specific wearer behaviour. To this end, the remote database may be completed with all the measured pairs of values. But here, only some of the pairs are stored in the remote database, for instance the ones that are the most illustrating of the wearer behaviour or, in a preferred embodiment, the ones for which we are the surest of their labelling (a threshold is for instance used to determine if a pair has to be stored or not).

In practice, the filling of the remote database is useful only during a limited period: after a given period, the filling of the remote database will not have any impact on the quality of the remote model.

For instance, when the pair of eyeglasses is initially sold or lent to the wearer, the database can comprise between 10 000 and 20 000 registers (preferably issued from values measured on at least five distinct wearers - here seven - and the more, the better). Then, the database is completed by a number of new registers lying between 10 000 and 20 000. In other words, the register initially stored in the database have less and less weights, but they are important to initiate the process and to label the new registers. Besides, a variant could consist of giving greater weight to the new registers than to the initial registers, or in removing little to little some of the initial registers.

In a third example, during the sale operations of the pair of eyeglasses 110, the processing unit 141 is programmed to carry out a learning operation during a learning period. More precisely, the future glasses wearer is asked by the optician to put on and then take off his pair of eyeglasses several times and under different conditions (by performing distinct movements, at different speeds...). Concurrently, pairs of values are measured and labelled by the optician, for instance thanks to a button that he presses when the pair of eyeglasses is worn and that he releases else, and they are then sent to the remote database. In Figure 3, curves F1, F2, F3, F4 illustrates the variations of four values (in this figure, it is considered that the frame is equipped with four distinct sensors), and the periods Δ represent the moments when the button is pressed. This learning period preferably lasts between 2 and 20 minutes, and more preferably between 4 and 7 minutes. Thanks to this learning operation, the remote database can be filled with values as representative as possible of the specific wearer behavior and the remote model can be adjusted as a function of these values.

In a fourth example, both second and third examples are combined. In other words, at the moment of the sale of the pair of eyeglasses 110, a learning operation is carried out and after, during at least a first part of the life of the eyeglasses, the database is completed with relevant data. This fourth example is, among all these examples, the one that gives the best results.

At the end of step S4, the last pair of values is associated to a worn or not-worn state. Then, a fifth step S5 is performed only if the pair of eyeglasses 110 is worn. This fifth step here consists in incrementing a wearing time counter when both sensors indicate that the pair of eyeglasses 110 is worn by the wearer.

After, steps S6 and S7 are performed. During these final steps S6 and S7, both sensors 142, 143 are shut down.

On the basis of the counter, it will be possible to perform many functions.

For instance, the length of time the pair of eyeglasses 110 has been worn during a predetermined period can be calculated and sent to the informatics device for next uses.

To this end, at the end of each day, it is possible to acquire the value of this counter, and to multiplicate this value by the time interval (here 30 seconds) in order to obtain the length of time the pair of eyeglasses 110 has been worn during the day, in seconds.

Then, the counter is reset (before the beginning of the following day).

The present invention is in no way limited to the embodiments described above and shown.

In a variant, other algorithms could be used instead of the k-means method. For instance, a Gaussian mixture model can be used, or a model based on a gradient-boosting decision tree. We could also use a neuronal network algorithm trained on the basis of a training database. In a preferred embodiment, the used method is the one that gives the best results. To this end, a batch of tests can be performed to determine, based on the kind of sensors embedded in the frame, the model that gives the best results.

In another variant, it could also be possible to fit the processing unit with several models and to select one model as a function of the conditions (for instance, if one of the sensors is a temperature sensor, it is possible to select a first model when the ambient temperature is close to 37°C, and another model else...). In this variant, the inputs of the models (that is to say the measured values) could be the same or distinct (a first model being based on the measures made by a first and a second sensors, and a second model being based on the measures made by a third and a fourth sensors).

In a second embodiment, the variation of the measured data during time can be taken into account to determine whether the pair of eyeglasses is worn or not. In the main embodiment disclose above, it is indeed the last pair of values that is considered. On the contrary, in this second embodiment, the processing unit is programmed to consider at least two last pair of measured values.

For instance, the processing unit acquires the last pairs of values determined during the last j sampling periods by the two sensors (with j at least equal to two, and here equal to five). Then, it calculates the standards deviations or the rolling means (also called "moving averages") of these values. The statistically obtained pair of value is then considered to determine whether it is nearer from one or the other cluster, and by deducing therefrom the worn state. This second embodiment gives better results than the main one because it is less dependent from measure errors.

This second embodiment can be carried out with any of the supervised and non-supervised method described above.

This second embodiment can also be carried out if the eyeglass frame comprises only one sensor and if, at each sampling period, a single value is measured. This variant of the second embodiment can be detailed.

In this variant, each time a measure is performed, this measure is registered in the database. During the first operation of clustering, the processing unit generates two cluster by considering each time j consecutive measures (here j=5). In other words, in this variant, a 5-uple of measures form an "observation" and the clustering is performed on the basis of all the recorded observations. Then, during the second operation, the processing unit reads the last j measures, and it determines whether this last observation is nearer from one or the other cluster, and it deduces therefrom the worn state.

We can also provide other variants of the invention.

For instance, the processing unit can modify the sampling period so as to reduce the energy consumption of the device or to obtain more detailed results.

In another example, the processing unit can affect distinct weights to the values measured by each sensor.

The application of the pair of eyeglasses 110 in the above embodiments is to detect the worn time for reducing myopia evolution. Other applications are possible, for instance for amblyopia risk prevention, for progressive addition lenses...

In another variant, the wearable device fitted with the wearing detection module could be of any other kind. It could be for instance a shoe for a person having legs of different lengths.

## Claims

1. Process for determining the wearing status of a wearable device (120) performed by a processing unit (141), said process comprising steps of:
- measuring the value of at least one physical parameter suitable for estimating whether the wearable device (120) is worn by a wearer or not, each measured value forming a n-tuple with n greater than or equal to 1, and
- determining if the wearable device (120) is worn as a function of said n-tuple,
**characterized in that** said step of determining is carried out thanks to a machine learning model.

2. Process according to claim 1, wherein, several n-tuples of values of said physical parameters being stored in a database and being classified in at least two clusters by means of said machine learning model, during the step of determining, it is determined if the measured n-tuple belongs to one or another of said clusters and it is deduced therefrom whether the wearable device (120) is worn or not.

3. Process according to claim 1 or 2, wherein several n-tuples of values of said at least one physical parameter are measured during time, and it is determined whether the wearable device (120) is worn or not as a function of the variation of said values during time.

4. Process according to claim 3, wherein a rolling mean or standard deviation of the values of at least one of said physical parameters is calculated and it is determined whether the wearable device (120) is worn or not as a function of the variation of said rolling mean or standard deviation.

5. Process according to any one of claims 1 to 4, wherein the measured n-tuple is stored in said database and wherein a clustering algorithm is then performed by said processing unit (141) on all the n-tuples of values stored in said database to generate new clusters.

6. Process according to claim 5, wherein said machine learning model is based on a K-means method or a gaussian mixture method.

7. Process according to any one of claim 1 to 6 combined with claim 2, wherein said n-tuples of values are classified in at least three clusters associated to a worn state, a non-worn state, and a worn but non-fitted state.

8. Process according to any one of claim 1 to 7, wherein n is greater than or equal to 2 and wherein at least two of said physical parameters are not of the same nature.

9. Process according to any one of claim 1 to 8, wherein n is greater than or equal to 2 and wherein at least two of said physical parameters are of the same nature.

10. Process according to any one of claim 1 to 9, wherein said parameter relates to:
- a position of a temple of the frame relative to another part of the frame, or
- a contact area or a distance between the wearable device (120) and the wearer, or
- a light emitted and reflected back by the wearer head, or
- a temperature on an internal face of the wearable device (120), or
- an inclination of the wearable device (120) relative to the ground.

11. Process according to any one of claim 1 to 10, wherein said machine learning model is trained on the basis of a database that stores, in registers, n-tuples of values and, associated to each n-tuple, a wearing state, said database being initially filled with registers issued from values measured on individuals others than said wearer and being then completed with registers issued from values measured on said wearer.

12. Wearing detection module (140) configured to be fixed on a wearable device (120), comprising:
- several wearing sensors (142, 143) configured to measure the value of at least one physical parameters suitable for estimating whether the wearable device (120) is worn by a wearer or not, each measured value forming a n-tuple with n greater than or equal to 1 and
- a processing unit (141) programmed to receive said n-tuple and to determine whether the wearable device (120) is worn or not, as a function of said n-tuple,
**characterized in that** said processing unit (141) is programmed to determine whether the wearable device (120) is worn or not thanks to a machine learning model.

13. Wearing detection module (140) according to claim 12, wherein distinct n-tuples of values of said physical parameters being measured during time, the processing unit (141) is programmed to determine whether the wearable device (120) is worn or not as a function of the variation of said values during time.

14. Wearing detection module (140) according to claim 12 or 13, wherein, n-tuples of values of said parameters being stored in a database and being classified in at least two clusters, the processing unit (141) is programmed to determine whether the wearable device (120) is worn or not by determining if the measured n-tuple belongs to one or another of said clusters.

15. Wearing detection module (140) according to any one of claims 12 to 14, wherein, when the wearing detection module (140) is new and ready to be sold, said database already stores several n-tuples of values of said parameters.

16. Wearing detection module (140) according to any one of claims 12 to 15, comprising a battery (144) configured to power the processing unit (141) and the wearing sensors (142, 143).

17. Monitoring equipment (100) comprising:
- a wearable device (120), and
- a wearing detection module (140) according to any one of claims 12 to 16, fixed on said frame.

18. The monitoring equipment (100) according to claim 17, wherein said wearable device (120) is a frame of an eyewear (110) that includes at least one lens (130) that is fixed to the frame and that is able to change the natural evolution of an optical deficiency.

19. The monitoring equipment (100) according to claim 17 or 18, wherein said wearable device (120) is a frame of an eyewear (110) and wherein said wearing sensors (142, 143) are embedded in a temple (122) of said frame.
